# EUROPEAN PATENT SPECIFICATION

(11) **EP 2 448 910 B1**
(45) Date of publication and mention of the grant of the patent: **15.05.2013**
(21) Application number: 10740144.0
(22) Date of filing: 28.06.2010
(51) Int. Cl.: C07C 209/62, C07C 209/16, C07C 211/35

(54) **METHOD OF PREPARING NERAMEXANE**
Verfahren zur Herstellung von Neramexan
Procédé de préparation de néramexane

(30) Priority: 29.06.2009 EP 09008466; 29.06.2009 US 269766 P
(43) Date of publication of application: 09.05.2012
(73) Proprietor: Merz Pharma GmbH & Co. KGaA, 60318 Frankfurt Main (DE)
(72) Inventor: KOLLER, Herbert, A-1100 Wien (AT); PYERIN, Michael, 2345 Brunn am Gebirge (AT); SBROGIÒ, Federico, 36075 Montecchio Maggiore (IT)
(74) Representative: Ricker, Mathias
(86) International application number: PCT/EP2010/003921
(87) International publication number: WO 2011/000538

(56) References cited:
- DANYSZ W ET AL: "AMINO-ALKYL-CYCLOHEXANS AS A NOVEL CLASS OF UNCOMPETITIVE NMDA RECEPTOR ANTAGONISTS" CURRENT PHARMACEUTICAL DESIGN, BENTHAM SCIENCE PUBLISHERS, NL, vol. 8, no. 10, 1 January 2002 (2002-01-01), pages 835-843, XP008030349 ISSN: 1381-6128 cited in the application

## Description

### FIELD OF THE INVENTION

This invention relates to a method of preparing 1-amino-1,3,3,5,5-pentamethylcyclohexane (Neramexane) or a pharmaceutically acceptable salt thereof.

### BACKGROUND OF THE INVENTION

1-amino-1,3,3,5,5-pentamethylcyclohexane (Neramexane) and pharmaceutically acceptable salts thereof are valuable agents for the continuous therapy of patients suffering from diseases and conditions such as tinnitus, and nystagmus.

Methods of preparing these agents are known.

In one method, commercially available isophorone is converted to Neramexane in a reaction sequence comprising five steps according to the following reaction scheme (W. Danysz et al., Current Pharmaceutical Design, 2002, 8, 835-843):

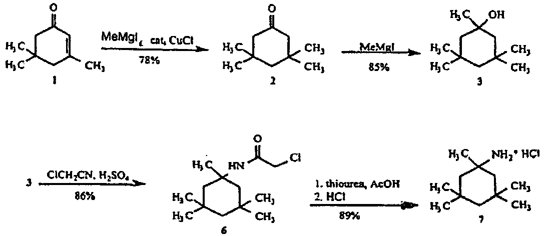

In the first step of the sequence, isophorone **1** is converted to 3,3,5,5-tetramethylcyclohexanone **2** by CuCl-catalyzed conjugate addition of methylmagnesium iodide.

Danysz discloses that compound **2** has been prepared according to the method of reference [3] (Kharasch). This reference discloses the reaction of isophorone with methylmagnesium bromide to the corresponding cyclohexanone. Page 2313, left column, discloses the "Addition of Isophorone to Methylmagnesium Bromide in the Presence of Cuprous Chloride". Compound **2** is characterized by the boiling point b.p. at two different pressures, by the melting point m.p., by refractory index n, by density d, by polarizability M, i.e. it must have been subjected to a step of purification such as distillation. Page 2313, right column, discloses the "The Addition of "Isophorone to Methylmagnesium Bromide in the Presence of Nickelous Chloride", wherein the target compound is isolated by fractionated distillation using a Vigreux column. Accordingly, compound **2** as used by Danysz is a purified product.

In the second step, 3,3,5,5-tetramethylcyclohexanon **2** is converted to 1,3,3,5,5-pentamethylcyclohexanol **3** by Grignard reaction with methylmagnesium iodide.

Danysz discloses that compound **3** has been prepared according to the method of reference [4] (Chiurdoglu). This reference discloses the reaction of 3,3,5,5-tetramethylcyclohexanone with methylmagnesium bromide to compound **3**. Page 377, section 5, discloses that the target compound has been subjected to distillation (boiling point 91 to 92 °C at 22 torr), i.e. it has been purified. Accordingly, compound **3** as used by Danysz is a purified product.

In the third step, said cyclohexanol **3** is converted to 1-chloroacetamido-1,3,3,5,5-pentamethylcyclohexane **6** by chloroacetonitrile in a Ritter reaction.

Danysz discloses that compound **6** has been prepared according to the method of reference [6] (Jirgensons). This reference discloses the Ritter reaction of the cyclohexanol with chloroacetonitrile to the respective amide according to step (iii) (Scheme on page 1709, compound 1a, compound 2a). According to the general reaction procedure, the resulting amide is subjected to a Kugelrohr short path distillation, i.e. it has been subjected to a purification step (page 1710, right column, first and second paragraph). Accordingly, compound **6** as used by Danysz is a purified product.

In the fourth step, subsequent cleavage of the chloroacetamido group in amide **6** with thiourea, and acidification of the resulting amine with hydrochloric acid in the final fifth step of the reaction sequence results in Neramexane (1-amino-1,3,3,5,5-pentamethylcyclohexane) **7** in the form of its hydrochloride.

The reported overall yield over the five steps of the reaction sequence is approximately 50 % by weight.

### OBJECTS OF THE INVENTION

One object of the invention is to improve one or more of the individual reaction steps of the above referenced reaction sequence in order to provide a method of preparing 1-amino-1,3,3,5,5-pentamethylcyclohexane or a pharmaceutically acceptable salt thereof that allows an advantageous realization on an economical industrial scale. It is in another object to minimize the amount of waste and/or unused chemicals produced during the manufacture of Neramexane or a pharmaceutically acceptable salt thereof. It is a further object to optimize or improve the yield and/or selectivity and/or product quality in regard to Neramexane or a pharmaceutically acceptable salt thereof. Such an improved method may be regarded as one prerequisite for an advantageous manufacture of Neramexane or a pharmaceutically acceptable salt thereof on an economical industrial scale.

### SUMMARY OF THE INVENTION

tee present invention relates to a method of preparing 1-amino-1,3,3,5,5-pentamethylcyclohexane or a pharmaceutically acceptable salt thereof, comprising at least steps (i) to (iv):
(i) converting isophorone to 3,3,5,5-tetramethylcyclohexanone;
(ii) converting 3,3,5,5-tetramethylcyclohexanone obtained in step (i) to 1-hydroxy-1,3,3,5,5-pentamethylcyclohexane;
(iii) converting 1-hydroxy-1,3,3,5,5-pentamethylcyclohexane obtained in step (ii) to 1-chloroacetamido-1,3,3,5,5-pentamethylcyclohexane;
(iv) converting 1-chloracetamido-1,3,3,5,5-pentamethylcyclohexane obtained in step (iii) to 1-amino-1,3,3,5,5-pentamethylcyclohexane;
wherein at least one of 3,3,5,5-tetramethylcyclohexanone, 1-hydroxy-1,3,3,5,5-pentamethylcyclohexane, 1-chloroacetamido-1,3,3,5,5-pentamethylcyclohexane, is not subjected to a purification step.

In one embodiment, the conversion in step (i) is effected by the reaction of isophorone with a methylmagnesium chloride in the presence of a copper(I) halide and a lithium halide.

In one embodiment, the conversion in step (ii) is effected by the reaction of 3,3,5,5-tetramethylcyclohexanone with a methylmagnesium chloride.

In one embodiment, the conversion in step (iii) is effected by the reaction of 1-hydroxy-1,3,3,5,5-pentamethylcyclohexane with chloroacetonitrile in acidic solution.

In one embodiment, the conversion in step (iv) is effected by reacting a mixture comprising 1-chloroacetamido-1,3,3,5,5-pentamethylcyclohexane, thiourea and water.

In one embodiment, the method further comprises step (v):
(v) converting 1-amino-1,3,3,5,5-pentamethylcyclohexane as obtained in step (iv) to a pharmaceutically acceptable salt thereof.

In one embodiment, the conversion in step (v) is effected by the reaction of 1-amino-1,3,3,5,5-pentamethylcyclohexane with an acid.

In one embodiment, the acid is methane sulphonic acid.

In one embodiment, the method comprises:
(i) converting isophorone to 3,3,5,5-tetramethylcyclohexanone in the presence of methylmagnesium chloride, copper(I) iodide, lithium chloride and tetrahydrofurane;
(ii) converting 3,3,5,5-tetramethylcyclohexanone as obtained in step (i) to 1-hydroxy-1,3,3,5,5-pentamethylcyclohexane in the presence of methylmagnesium chloride and tetrahydrofurane;
(iii) converting 1-hydroxy-1,3,3,5,5-pentamethylcyclohexane as obtained in step (ii) to 1-chloroacetamido-1,3,3,5,5-pentamethylcyclohexane in the presence of chloroacetonitrile, acetic acid and sulphuric acid;
(iv) converting 1-chloroacetamido-1,3,3,5,5-pentamethylcyclohexane as obtained in step (iii) to 1-amino-1,3,3,5,5-pentamethylcyclohexane in the presence of thiourea, water and hydrochloric acid.

In one embodiment, said methylmagnesium chloride is free of ethylmagnesium chloride.

It has unexpectedly been discovered that in the reaction sequence comprising steps (i) to (iv) according to the invention, the purification of one or more of 3,3,5,5-tetramethylcyclohexanone, 1-hydroxy-1,3,3,5,5-pentamethylcyclohexane, 1-chloroacetamido-1,3,3,5,5-pentamethylcyclohexane, by means of the classical purification methods such as distillation or recrystallization or chromatography may be omitted. Accordingly, one or more of said compounds as obtained in the respective steps (i) to (iii) is/are not subjected to a purification step and is/are employed in a non-purified form in the respective subsequent steps (ii) to (iv).

It could not be expected that by employing one or more of the non-purified intermediates, the target compound, i.e. Neramexane, or Neramexane in the form of a pharmaceutically acceptable salt, may be obtained in a purity that is sufficient for the medicinal application. Thus, since the method according to the invention allows the omission of complex cleaning steps of the intermediates such as distillation or recrystallization or chromatography, which commonly result in product loss, a yield of Neramexane or a pharmaceutically acceptable salt thereof of at least 60 % by weight is possible. Accordingly, the novel simplified method of producing Neramexane may be performed on an advantageous economical industrial scale.

### DETAILED DESCRIPTION OF THE INVENTION

The present invention relates to a method of preparing 1-amino-1,3,3,5,5-pentamethylcyclohexane (Neramexane) or a pharmaceutically acceptable salt thereof.

Specifically, the present invention relates to a method of preparing 1-amino-1,3,3,5,5-pentamethylcyclohexane or a pharmaceutically acceptable salt thereof, comprising at least steps (i) to (iv):
(i) converting isophorone to 3,3,5,5-tetramethylcyclohexanone;
(ii) converting 3,3,5,5-tetramethylcyclohexanone as obtained in step (i) to 1-hydroxy-1,3,3,5,5-pentamethylcyclohexane;
(iii) converting 1-hydroxy-1,3,3,5,5-pentamethylcyclohexane as obtained in step (ii) to 1-chloroacetamido-1,3,3,5,5-pentamethylcyclohexane;
(iv) converting 1-chloroacetamido-1,3,3,5,5-pentamethylcyclohexane as obtained in step (iii) to 1-amino-1,3,3,5,5-pentamethylcyclohexane;
wherein at least one of 3,3,5,5-tetramethylcyclohexanone, 1-hydroxy-1,3,3,5,5-pentamethylcyclohexane, 1-chloroacetamido-1,3,3,5,5-pentamethylcyclohexane, is not subjected to a purification step.

Accordingly, the method according to the invention includes that at least one of the compounds 3,3,5,5-tetramethylcyclohexanone, 1-hydroxy-1,3,3,5,5-pentamethylcyclohexane, 1-chloroacetamido-1,3,3,5,5-pentamethylcyclohexane, is employed in the corresponding reaction step just in the same form as it has been obtainedin the previous step of the reaction sequence, i.e. without subjecting the at least one compound prepared in the sequence of steps (i) to (iii) to a purification step.

The term *"purification step"* encompasses the recrystallization, distillation, or chromatography, or combinations thereof, of the compound yielded in the respective reaction step (i) to (iii), i.e. one of 3,3,5,5-tetramethylcyclohexanone, 1-hydroxy-1,3,3,5,5-pentamethylcyclohexane, 1-chloroacetamido-1,3,3,5,5-pentamethyl-cyclohexane.

The term *"is not subjected to a purification step"* allows standard work up steps such as the removing of a solvent from a mixture comprising said compound, i.e. said 3,3,5,5-tetramethylcyclohexanone, 1-hydroxy-1,3,3,5,5-pentamethylcyclohexane, 1-chloroacetamido-1,3,3,5,5-pentamethylcyclohexane, and said solvent by distillation, or the extraction of said compound from an aqueous phase by means of a solvent, or the drying of a mixture comprising said compound and a solvent using e.g. anhydrous sodium sulphate, the drying of said compound in vacuo, the washing of a solid compound with a liquid, and the like.

Purification by *"recrystallization", "distillation",* or *"chromatography"* are the classical methods employed for purifying chemical compounds such as organic compounds both on a laboratory and an industrial scale.

Recrystallization is a method of separating mixtures based on differences of the compounds contained therein in their solubilities in a solvent or a mixture of solvents. If a compound is to be purified by recrystallization, it is dissolved in an appropriate solvent, which is then allowed to cool. This results in the desired purified compound dropping (recrystallization) from the solution. However, it is also possible to add to the solution another solvent, in which the desired compound is insoluble, until the desired compounds begins to precipitate. Accordingly, in the meaning of the present invention, the term *"recrystallization"* means that a compound (here: 3,3,5,5-tetramethylcyclohexanone, 1-hydroxy-1,3,3,5,5-pentamethylcyclohexane, 1-chloroacetamido-1,3,3,5,5-pentamethylcyclohexane) has to be transferred to a dissolved condition and precipitates or is precipitated from said dissolved condition to form the purified compound, which is isolated.

Distillation is a method of separating mixtures based on differences of the compounds contained therein in their volatilities in a boiling liquid mixture. Accordingly, in the meaning of the present invention, the term *"distillation"* as mentioned in the definition of the term *"purification"* means that a compound (here: 3,3,5,5-tetramethylcyclohexanone, 1-hydroxy-1,3,3,5,5-pentamethylcyclohexane, 1-chloroacetamido-1,3,3,5,5-pentamethylcyclohexane) has to be transferred from the liquid phase to the vapour phase and is subsequently condensed to form the purified compound, which is isolated.

Chromatography in chemistry is a method of separating mixtures based on differences in the distribution of the compounds contained therein between a stationary phase and a mobile phase. A typical method is column chromatography which may be used for preparative applications. Accordingly, in the meaning of the present invention, the term *"chromatography"* as mentioned in the definition of the term *"purification"* means that a compound (here: 3,3,5,5-tetramethylcyclohexanone, 1-hydroxy-1,3,3,5,5-pentamethylcyclohexane, 1-chloroacetamido-1,3,3,5,5-pentamethylcyclohexane) has to be distributed between a stationary phase and a mobile phase to form the purified compound, which is isolated.

Accordingly, at least one of 3,3,5,5-tetramethylcyclohexanone, 1-hydroxy-1,3,3,5,5-pentamethylcyclohexane, 1-chloroacetamido-1,3,3,5,5-pentamethylcyclohexane as prepared in the sequence of step (i) to step (iii) is not subjected to any of the above defined purification steps, and is employed in the respective subsequent steps (ii) to (iv) without employing said purification steps.

Thus, in one embodiment of the method according to the invention, one of 3,3,5,5-tetramethylcyclohexanone, 1-hydroxy-1,3,3,5,5-pentamethylcyclohexane, 1-chloroacetamido-1,3,3,5,5-penta-methylcyclohexane, is not subjected to a purification step of recrystallization or distillation or chromatography.

In one embodiment, 3,3,5,5-tetramethylcyclohexanol as obtained in step (i) is not subjected to a purification step.

At ambient temperature (25 °C), 3,3,5,5-tetramethylcyclohexanone as obtained in step (i) and employed in step (ii) is a liquid.

In one embodiment, said compound is not subjected to distillation. This means that 3,3,5,5-tetramethylcyclohexanone is not transferred from the liquid phase to the vapour phase and is subsequently condensed to form the purified compound.

In another embodiment, 3,3,5,5-tetramethylcyclohexanone is not distributed between a stationary phase and a mobile phase to form the purified compound.

In another embodiment, 3,3,5,5-tetramethylcyclohexanone is not transferred to a dissolved condition and precipitates or is precipitated from said dissolved condition to form the purified compound.

In another embodiment, 1-hydroxy-1,3,3,5,5-pentamethylcyclohexane as obtained in step (ii) is not subjected to a purification step.

At ambient temperature, 1-hydroxy-1,3,3,5,5-pentamethylcyclohexane as obtained in step (ii) and employed in step (iii) is a liquid.

In one embodiment, said compound is not subjected to distillation. This means that 1-hydroxy-1,3,3,5,5-pentamethylcyclohexane is not transferred from the liquid phase to the vapour phase and is subsequently condensed to form the purified compound.

In another embodiment, 1-hydroxy-1,3,3,5,5-pentamethylcyclohexane is not distributed between a stationary phase and a mobile phase in order to purify the compound.

In another embodiment, 1-hydroxy-1,3,3,5,5-pentamethylcyclohexane is not transferred to a dissolved condition and precipitates or is precipitated from said dissolved condition to form the purified compound.

In one embodiment, 1-chloroacetamido-1,3,3,5,5-pentamethylcyclohexane as obtained in step (iii) is not subjected to a purification step.

At ambient temperature, 1-chloroacetamido-1,3,3,5,5-pentamethylcyclohexane as obtained in step (iii) and employed in step (iv) is a solid.

In one embodiment, said compound is not subjected to recrystallization. This means that 1-chloroacetamido-1,3,3,5,5-pentamethylcyclohexane is not transferred to a dissolved condition and precipitates or is precipitated from said dissolved condition to form the purified product.

In another embodiment, 1-chloroacetamido-1,3,3,5,5-pentamethylcyclohexane is not distributed between a stationary phase and a mobile phase in order to form the purified the compound.

In another embodiment, 1-chloroacetamido-1,3,3,5,5-pentamethylcyclohexane is not subjected to distillation. This means that 1-chloroacetamido-1,3,3,5,5-pentamethylcyclohexane is not transferred from the liquid phase to the vapour phase and is subsequently condensed to form the purified compound.

In one embodiment, 3,3,5,5-tetramethylcyclohexanone as obtained in step (i) and 1-hydroxy-1,3,3,5,5-pentamethylcyclohexane as obtained in step (ii) are not subjected to a purification step.

In another embodiment, 3,3,5,5-tetramethylcyclohexanone as obtained in step (i), 1-hydroxy-1,3,3,5,5-pentamethylcyclohexane as obtained in step (ii) and 1-chloroacetamido-1,3,3,5,5-pentamethylcyclohexane as obtained in step (iii) are not subjected to a purification step.

In one embodiment, 1-hydroxy-1,3,3,5,5-pentamethylcyclohexane as obtained in step (ii) and 1-chloroacetamido-1,3,3,5,5-pentamethylcyclohexane as obtained in step (iii) are not subjected to a purification step.

In another embodiment, 3,3,5,5-tetramethylcyclohexanone as obtained in step (i), and 1-chloroacetamido-1,3,3,5,5-pentamethylcyclohexane as obtained in step (iii) are not subjected to a purification step.

*Conversion of isophorone to 3,3,5,5-tetramethylcyclohexanone (step (i))*

In one embodiment of the method according to the invention, the conversion in step (i) is effected by the reaction of isophorone with a methylmagnesium halide.

In one embodiment, the methylmagnesium halide is selected from the group consisting of methylmagnesium iodide, methylmagnesium bromide and methylmagnesium chloride.

Such Grignard reagents may be produced from magnesium and the respective methyl halide.

In one embodiment, the conversion in step (i) is performed in the presence of a copper compound. Said copper compound may serve as a catalyst in order to benefit the conjugate 1,4-addition of the Grignard reagent to isophorone over the 1,2-addition. In one embodiment, the copper compound is a copper(I) halide.

In one embodiment, the copper(I) halide is selected from the group consisting of copper(I) iodide, copper(I) bromide or copper(I) chloride.

In one embodiment, said copper compound (e.g. copper(I) halide such as copper(I) chloride or copper(I) iodide) is provided in the presence of a lithium compound.

In one embodiment the lithium compound is a lithium halide such as lithium chloride.

In one embodiment, copper(I) chloride or copper(i) iodide is provided in the presence of lithium chloride.

In one embodiment, said methylmagnesium halide is methylmagnesium chloride and the copper(I) halide is copper(I) chloride or copper (I) iodide.

In still another embodiment, the methylmagnesium halide is methylmagnesium chloride and the copper(I) halide is copper(I) iodide.

In still another embodiment, the conversion in step (i) is effected by the reaction of isophorone with methylmagnesium chloride in the presence of copper(I) iodide or copper(I) chloride and lithium chloride.

In one embodiment, the molar ratio of copper(I) halide to lithium halide is in the range of from 1:1.5 to 1:2.5.

In one embodiment, the ratio of copper(I) chloride or copper(I) iodide to lithium chloride is about 1 : 1.5 to 1 : 2.5, or 1 : 2, respectively.

The reaction according to step (i) commonly is performed in a solvent.

In one embodiment, the solvent employed for the reaction in step (i) is an ether, or the solvent comprises an ether.

Suitable ethers may be selected from the group consisting of diethyl ether, 1,4-dioxane, tetrahydrofurane.

In one embodiment, said ether is tetrahydrofurane.

In one embodiment, the solvent employed in step (i) comprises tetrahydrofurane or is tetrahydrofurane.

In one embodiment, isophorone is converted to 3,3,5,5-tetramethylcyclohexanone by using methylmagnesium chloride, copper(I) chloride or copper (I) iodide and lithium chloride in tetrahydrofurane.

In one embodiment, isophorone is converted to 3,3,5,5-tetramethylcyclohexanone by using methylmagnesium chloride, copper (I) iodide and lithium chloride in tetrahydrofurane.

In one embodiment, isophorone, the copper compound such as copper (I) halide (e.g. copper(I) iodide or copper(I) chloride) and, optionally, the lithium compound such as lithium halide (e.g. lithium chloride), are provided in a solvent, and the Grignard reagent, optionally dissolved in a solvent, is added to said mixture.

In one embodiment, methylmagnesium chloride is dissolved in tetrahydrofurane.

In one embodiment, the concentration of methylmagnesium chloride in tetrahydrofurane is from 15 to 30 % by weight, or 20 to 25 % by weight based on the total amount of methylmagnesium chloride and tetrahydrofurane.

In one embodiment, the concentration of methylmagnesium chloride in tetrahydrofurane is 23 % by weight based on the total amount of methylmagnesium chloride and tetrahydrofurane.

In one embodiment, more than one molar equivalent methylmagnesium chloride are employed per one molar equivalent isophorone.

In one embodiment, from 1.0 to 1.75 molar equivalents methylmagnesium chloride, or from 1.2 to 1.5 molar equivalents methylmagnesium chloride are employed per one molar equivalent isophorone.

In one embodiment, the concentration of methylmagnesium chloride in tetrahydrofurane is 23 % by weight based on the total amount of methylmagnesium chloride and tetrahydrofurane, and 10 % by weight catalyst (one molar equivalent copper(I) iodide and two molar equivalents lithium chloride) based on the amount of methylmagnesium chloride and tetrahydrofurane are employed.

In one embodiment, from 0.1 to 0.25 molar equivalents lithium chloride and from 0.05 to 0.125 molar equivalents copper(I) iodide per one molar equivalent isophorone are employed.

In another embodiment, methylmagnesium chloride is reacted with the copper compound such as a copper(I) halide (e.g. copper (I) iodide or copper(I) chloride), optionally in the presence of a lithium compound such as lithium halide (e.g. lithium chloride). In one embodiment, said mixture is added to isophorone. In another embodiment, isophorone is added to said mixture.

In another embodiment, methylmagnesium chloride is reacted with a copper compound such as copper(I) iodide or copper(I) chloride.

In one embodiment, a mixture of isophorone, copper (I) iodide and lithium chloride is provided in tetrahydrofurane. Methylmagnesium chloride which is dissolved in tetrahydrofurane, is added to said mixture.

The addition is performed such that the temperature can be controlled.

In one embodiment, the addition is performed such that the temperature may be maintained in a relatively narrow temperature range.

In one embodiment, the conversion in step (i) is performed at a temperature of from -5 °C to 20 °C, or 0 °C to 20 °C, or -5 °C to 15 °C, or -1 °C to 10 °C.

The reaction between the Grignard reagent and isophorone commonly proceeds rather fast. Usually, the reaction may be terminated after three hours or two hours or even one hour, depending on the reaction temperature employed.

After the reaction of isophorone with the Grignard reagent, the reaction mixture may be treated with water in order to destroy an excess of Grignard reagent, if any employed, respectively to destroy basic magnesium compounds.

In one embodiment, an acid such as hydrochloric acid or an ammonium salt is added to support the formation of 3,3,5,5-tetramethylcyclohexanone.

In one embodiment, the product formed in step (i) is obtained and isolated by extracting the aqueous mixture with an appropriate organic solvent such as methylene chloride or toluene or petroleum ether. Subsequent to extracting, the solvent is removed by distillation. The liquid residue comprising crude 3,3,5,5-tetramethylcyclohexanone as obtained and isolated may be employed without purification in step (ii) of the reaction sequence. Accordingly, 3,3,5,5-tetramethylcyclohexanone is not subjected to a distillation step, i.e. is not transferred from the liquid phase to the vapour phase and is subsequently condensed to form the purified compound.

In another embodiment, subsequent to extracting, the extract may be dried according to known methods. For example, the extract may be dried over sodium sulphate. After separating off said sulphate by filtration, the solvent may be removed by distillation. The residue comprising crude *3,3,5,5-tetramethylcyclohexanone* as obtained and isolated may be employed without purification in step (ii) of the reaction sequence. Accordingly, *3,3,5,5-tetramethylcyclohexanone* is not subjected to a distillation step, i.e. is not transferred from the liquid phase to the vapour phase and is subsequently condensed to form the purified compound.

In one embodiment, the yield of crude *3,3,5,5-tetramethylcyclohexanone* as obtained and isolated in step (i) is in the range of from 88 % to 96 % by weight.

In one embodiment, the crude product contains the target compound in an amount of at least 93 % by weight and less than 99 % by weight as can be determined by gas-liquid chromatography.

*Conversion of 3,3,5,5-tetramethylcyclohexanone to 1-hydroxy-1,3,3,5,5-*pentamethylcyclohexane *(step (ii))*

In one embodiment, the conversion of 3,3,5,5-tetramethylcyclohexanone to 1-hydroxy-1,3,3,5,5-pentamethylcyclohexane in step (ii) is effected with a methylmagnesium halide.

As methylmagnesium halide, the iodide, bromide or chloride may be used.

In one embodiment, said methylmagnesium halide is methylmagnesium chloride.

The reaction according to step (ii) commonly is performed in a solvent.

In one embodiment, said solvent comprises an ether, or the solvent is an ether.

Ethers may be selected from diethyl ether, 1,4-dioxane, or tetrahydrofurane.

In one embodiment, said ether is tetrahydrofurane.

In one embodiment of the method of the invention, methylmagnesium chloride is added to 3,3,5,5-tetramethylcyclohexanone.

In another embodiment, tetramethylcyclohexanone is added to methylmagnesium chloride.

In one embodiment, a solution of methylmagnesium chloride in tetrahydrofurane is added to a solution of 3,3,5,5-tetramethylcyclohexanone in tetrahydrofurane.

In another embodiment, a solution of 3,3,5,5-tetramethylcyclohexanone in tetrahydrofurane is added to a solution of methylmagnesium chloride in tetrahydrofurane.

Accordingly, in one embodiment, a mixture comprising methylmagnesium chloride and tetrahydrofurane is reacted with a mixture comprising 3,3,5,5-tetramethylcyclohexanone and tetrahydrofurane.

In one embodiment, more than one molar equivalent methylmagnesium chloride is employed per molar equivalent 3,3,5,5-tetramethylcyclohexanone as obtained in step (i), such as 1.1 to 2.0 molar equivalents.

In one embodiment, about 1.2 to 1.75 molar equivalents methylmagnesium chloride are employed per molar equivalent 3,3,5,5-tetramethylcyclohexanone as obtained in step (i).

In one embodiment, a solution of 3,3,5,5-tetramethylcyclohexanone as obtained in step (i) in tetrahydrofurane is added to a solution of methylmagnesium chloride in tetrahydrofurane.

In one embodiment, a solution of 3,3,5,5-tetramethylcyclohexanone in tetrahydrofurane is added to a solution of methylmagnesium chloride in tetrahydrofurane, which contains from 1.2 to 1.75 molar equivalents methylmagnesium chloride per molar equivalent 3,3,5,5-tetramethylcyclohexanone.

In one embodiment, about 1.2 to 1.75 molar equivalents methylmagnesium chloride are employed per molar equivalent 3,3,5,5-tetramethylcyclohexanone obtained in step (i).

In another embodiment, a solution comprising methylmagnesium chloride in tetrahydrofurane is added to a solution comprising 3,3,5,5-tetramethylcyclohexanone as obtained in step (i) in tetrahydrofurane.

In one embodiment, the conversion is performed such that the temperature is controlled.

In one embodiment, the conversion is performed such that the temperature is maintained in a relatively narrow temperature range.

In one embodiment, the conversion in step (ii) is performed at a temperature of from -5 °C to 30 °C, or 0 °C to 30 °C, or 0 °C to 25 °C, or 0 °C to 20 °C, or 5 °C to 20 °C, or 10 °C to 25 °C, or 15 to 25 °C.

For isolating the formed 1-hydroxy-1,3,3,5,5-pentamethylcyclohexane in step (ii), basically the same methods may be employed as discussed above in connection with the isolation of 3,3,5,5-tetramethylcyclohexanone in step (i).

Accordingly, in one embodiment, 1-hydroxy-1,3,3,5,5-pentamethylcyclohexane as obtained in step (ii) is not subjected to a distillation step, i.e. is not transferred from the liquid phase to the vapour phase and is subsequently condensed to form the purified compound.

In one embodiment, the yield of crude 3,3,5,5-tetramethylcyclohexanone ranges between 90 % and 100 % by weight.

In one embodiment, the crude product contains the target compound 1-hydroxy-1,3,3,5,5-pentamethylcyclohexane in an amount of at least 94 % by weight and less than 99 % by weight as can be determined by gas-liquid chromatography.

*Conversion of 1-hydroxy-1,3,3,5,5-pentamethylcyclohexane to 1-chloroacetamido-1,3,3,5,5-pentamethylcyclohexane (step (iii))*

In one embodiment, the conversion in step (iii) is effected by means of the reaction of 1-hydroxy-1,3,3,5,5-pentamethylcyclohexane with chloroacetonitrile in acidic solution.

The Ritter reaction of step (iii) may be performed according to the methods as referenced in the prior art.

In one embodiment, 1-hydroxy-1,3,3,5,5-pentamethylcyclohexane as obtained in step (ii) and chloroacetonitrile are provided in acetic acid, and sulphuric acid is added to said mixture.

In another embodiment, 1-hydroxy-1,3,3,5,5-pentamethylcyclohexane as obtained in step (ii) is provided in acetic acid, and a mixture of chloroacetonitrile and sulphuric acid is added to said mixture.

In one embodiment, 1-hydroxy-1,3,3,5,5-pentamethylcyclohexane and acetic acid are provided in a weight ratio of from 1 : 1.5 to 1 : 2.5.

In one embodiment, said cyclohexanol and acetic acid are provided in a weight ratio of about 1 : 2.

In another embodiment, about 2 molar equivalents chloroacetonitrile and 3 molar equivalents sulphuric acid are employed.

In another embodiment, per molar equivalent 1-hydroxy-1,3,3,5,5-pentamethylcyclohexane, from 1.5 to 2.5 molar equivalents chloroacetonitrile and from 2.5 to 3.5 molar equivalents sulphuric acid are employed.

In one embodiment, 1-hydroxy-1,3,3,5,5-pentamethylcyclohexane and acetic acid are provided in a weight ratio of from 1 : 1.5 to 1 : 2.5; and, per molar equivalent 1-hydroxy-1,3,3,5,5-pentamethylcyclohexane, from 1.5 to 2.5 molar equivalents chloroacetonitrile and from 2.5 to 3.5 molar equivalents sulphuric acid are employed.

In one embodiment, said cyclohexanol and acetic acid are provided in a weight ratio of about 1 : 2; and 2 molar equivalents chloroacetonitrile and 3 molar equivalents sulphuric acid are employed per molar equivalent 1-hydroxy-1,3,3,5,5-pentamethylcyclohexane.

In one embodiment, the addition of sulphuric acid or the mixture of chloroacetonitrile and sulphuric acid is performed such that the reaction temperature is kept in a range of from 0 °C to 30 °C, or 0 °C to 20 °C, or 0 °C to 15 °C, or 5 °C to 10 °C.

In general, the reaction proceeds relatively fast towards the target compound. In one embodiment, the reaction may be terminated after 2 hours, or even one hour.

After the termination of the reaction, the reaction mixture may be poured into water or ice or ice and water in order to work up the mixture. The precipitating 1-chloroacetamido-1,3,3,5,5-pentamethylcyclohexane may be isolated by filtration.

The precipitate may be washed with water in order to remove adhering acid.

In one embodiment, the yield of crude product is in the range of from 98 to 100 % by weight.

Accordingly, in one embodiment, 1-chloroacetamido-1,3,3,5,5-pentamethylcyclohexane is not transferred to a dissolved condition and precipitates or is precipitated from said dissolved condition to form the purified compound.

*Conversion of 1-chloroacetamido-1,3,3,5,5-pentamethylcyclohexane to 1-amino-1,3,3,5,5-pentamethylcyclohexane (step (iv))*

In one embodiment, the conversion in step (iv) is effected by the reaction of 1-chloroacetamido-1,3,3,5,5-pentamethylcyclohexane with thiourea.

In one embodiment, the mixture employed in step (iv) comprises water.

In one embodiment, 1-chloroacetamido-1,3,3,5,5-pentamethylcyclohexane is reacted with thiourea in acetic acid as referenced in the Background section.

In one embodiment, 1-chloroacetamido-1,3,3,5,5-pentamethylcyclohexane as obtained in step (iii) and which is not subjected to a purification step may be employed in step (iv). The compound may be employed in dried form or in still humid form.

In one embodiment, the mixture employed in step (iv) further comprises an organic solvent.

In one embodiment, said organic solvent is a solvent that is miscible with water under the reaction conditions employed in step (iv), such as an alcohol.

In one embodiment, said organic solvent is an alcohol selected from the group consisting of methanol, ethanol, propanol, butanol, ethylene glycol.

In one embodiment, the amount of said organic solvent is from 0 to 200 % by weight based on the amount of water. In another embodiment, the amount of said organic solvent is from 0 to 150 % by weight, or from 0 to 100 % by weight, or from 0 to 50 % by weight, or from 0 to 10 % by weight, or from 0 to 5 % by weight based on the amount of water.

In another embodiment, the mixture as employed in step (iv), is substantially free from an organic solvent.

The term *"substantially free from an organic solvent"* envisions that the mixture contains said organic solvent in an amount of from 0 to 5 % by weight based on the amount of water, or from 0 to 3 % by weight, or from 0 to 1 % by weight.

In one embodiment, the weight ratio of thiourea to water is in the range of from 1 : 0.5 to 1 : 50, or from 1 : 1 to 1 : 20, or from 1 : 2 to 1 : 10.

Although the reaction according to step (iv) may be performed without the addition of an acid, the addition of such a compound may accelerate the conversion of 1-chloroacetamido-1,3,3,5,5-pentamethylcyclohexane to 1-amino-1,3,3,5,5-pentamethylcyclohexane.

Accordingly, in one embodiment, the mixture of step (iv) further comprises an acid.

Acids that may be employed are, but not limited to, hydrochloric acid, sulphuric acid, phosphorus acid, p-toluenesulphonic acid, methanesulphonic acid, acetic acid, benzoic acid. Accordingly, inorganic as well as organic acids may be used.

The amount of acid employed, if any, may be in a relatively broad range.

In one embodiment, the mixture comprises an acid in an amount of from 0.1 to 20 % by weight based on the amount of water.

In one embodiment, the acid employed is hydrochloric acid.

In order to further accelerate the conversion of 1-chloroacetamido-1,3,3,5,5-pentamethylcyclohexane, the mixture employed in step (iv) is heated, wherein the reaction proceeds.

The term *"heating"* includes that the mixture employed in step (iv) is set to a temperature above ambient temperature (25 °C).

In one embodiment, the mixture as employed in step (iv) is heated up to a temperature in the range of from 50 °C to the reflux temperature of the mixture.

In another embodiment, the mixture is heated up to a temperature in the range of from 80 °C to the reflux temperature of the mixture.

In still another embodiment, the mixture is heated up to the reflux temperature of the mixture.

If in step (iv) a mixture is employed that is substantially free from an organic solvent, the reflux temperature usually is around 100 °C, i.e. in the range of from 95 to 105 °C. If in step (iv) a mixture is employed that contains an organic solvent, the reflux temperature may be higher or lower than the reflux temperature of a mixture comprising water but that is substantially free from an organic solvent, depending on the amount and boiling point of the organic solvent employed.

The conversion of 1-chloroacetamido-1,3,3,5,5-pentamethylcyclohexane to 1-amino-1,3,3,5,5-pentamethylcyclohexane according to step (iv) may be controlled by the common chromatographical methods, e.g. by gas-liquid chromatography.

In one embodiment, in step (iv), 1.0 to 2 mole thiourea per 1 mole 1-chloroacetamido-1,3,3,5,5-pentamethylcyclohexane, 1 to 3 mole acid and 500 to 1,500 % by weight water based on the amount of thiourea and 1-chloroacetamido-1,3,3,5,5-pentamethylcyclohexane are employed at reflux temperature.

In one embodiment, one molar equivalent 1-choroacetamido-1,3,3,5,5-pentamethylcyclohexane is reacted with approximately 1.2 molar equivalents thiourea and 2 molar equivalents hydrochloric acid in the 8-fold amount of water (by weight based on thiourea and 1-choroacetamido-1,3,3,5,5-pentamethylcyclohexane) at reflux temperature.

Commonly, the conversion in the water-containing mixture of step (iv) proceeds rather fast.

In one embodiment, wherein step (iv) is performed in water that is substantially free from an organic solvent, and wherein the heating is performed at reflux temperature, i.e. at a temperature around 100 °C, and wherein an acid is added, the conversion may even be terminated after 2 hours, or even 1 hour.

In one embodiment, the conversion is terminated already after 6 hours, or 5 hours, or even four hours, or even 3 hours, or even less than 3 hours.

If the conversion is catalyzed by an acid, at least a part of the generated amine will be dissolved in water due to the protonation of the amino group, thus forming a salt.

In one embodiment, said mixture comprising 1-choroacetamido-1,3,3,5,5-pentamethylcyclohexane, thiourea, hydrochloric acid and water forms a homogeneous solution upon heating.

In one embodiment, in order to isolate the produced amine, the method of the invention further comprises the addition of alkali to the mixture to set the pH to a value of at least 7, and separating off 1-amino-1,3,3,5,5-pentamethylcyclohexane from the mixture.

In said embodiment, preferably after cooling the mixture down, the amine separates from the aqueous phase after the addition of alkali, and may be separated off.

In another embodiment, the amine may be extracted from the mixture which, after the addition of alkali, comprises an aqueous and an organic phase, with an organic solvent, which is not miscible with water. Suitable solvents are solvents such as methylene chloride, toluene or petroleum ether. Subsequent to the extraction, the extract may be dried using sodium sulphate or the like. After removing the solvent by evaporation, the crude amine is obtained.

In one embodiment, the yield of crude product is at least 95 % by weight of the theory, or even nearly quantitative. The crude product in general contains the target compound in a very high amount of more than 95 % by weight, or more than 97 % by weight, or even 99 % by weight as determined by gas-liquid chromatography.

In one embodiment, if necessary, the crude amine may be further purified by distillation.

The product as obtained and isolated in step (iv) may be employed without further purification in step (v) of the method according to the invention.

However, in one embodiment, it is also possible to distil off compounds from the crude product having a higher volatility than 1-amino-1,3,3,5,5-pentamethylcyclohexane, and to employ the residue in step (v).

In one embodiment, 1-amino-1,3,3,5,5-pentamethylcyclohexane is purified by distillation.

*Conversion of 1-amino-1,3,3,5,5-pentamethylcyclohexane to a salt of 1-amino-1,3,3,5,5-pentamethylcyclohexane (step (v))*

In one embodiment, in step (v), *1-amino-1,3,3,5,5-pentamethylcyclohexane* is converted into a pharmaceutically acceptable salt thereof by addition of an appropriate acid.

For the purpose of this disclosure, the term "*pharmaceutically acceptable* s*alts*" refers to salts of neramexane that are physiologically tolerable and do not typically produce untoward reactions when administered to a mammal (e.g., human). Typically, the term *"pharmaceutically acceptable salt"* means approved by a regulatory agency of the Federal or a state government or listed in the U.S. Pharmacopeia or other generally recognized pharmacopeia for use in mammals, and more particularly in humans.

Conversion of 1-amino-1,3,3,5,5-pentamethylcyclohexane to a pharmaceutically acceptable salt thereof is accomplished in conventional fashion by admixture of the base with at least one molecular equivalent of a selected acid in an inert organic solvent. Isolation of the salt is carried out by techniques known to the art such as inducing precipitation with a non-polar solvent (e.g. ether) in which the salt has limited solubility. The nature of the salt is not critical, provided that it is non-toxic and does not substantially interfere with the desired pharmacological activity.

Examples of pharmaceutically acceptable salts are those formed with hydrochloric, hydrobromic, methanesulfonic, acetic, succinic, maleic, citric acid, and related acids.

Further pharmaceutically acceptable salts include, but are not limited to, acid addition salts, such as those made with hydroiodic, perchloric, sulfuric, nitric, phosphoric, propionic, glycolic, lactic, pyruvic, malonic, fumaric, tartaric, benzoic, carbonic, cinnamic, mandelic, ethanesulfonic, hydroxyethanesulfonic, benezenesulfonic, p-toluene sulfonic, cyclohexanesulfamic, salicyclic, p-aminosalicylic, 2-phenoxybenzoic, and 2-acetoxybenzoic acid.

In one embodiment, prior to the addition of an acid, 1-amino-1,3,3,5,5-pentamethylcyclohexane as obtained in step (iv) is dissolved or dispersed or suspended in a solvent or a mixture of two or more solvents.

Suitable solvents are solvents such as acetone, anisole, butyl acetate, t-butylmethyl ether, cumene, dimethylsulphoxide, ethyl acetate, ethyl ether, ethyl formate, heptane, i-butyl acetate, i-propyl acetate, methyl acetate, methylethyl ketone, methyl-i-butyl ketone, pentane, propyl acetate, tetrahydrofurane, 1,1-diethoxypropane, 1,1-dimethoxymethane, 2,2-dimethoxypropane, isooctane, isopropyl ether, methyl-i-propyl ketone and methyltetrahydrofurane.

In one embodiment, a mixture of a solvent and water such as methylethyl ketone and water may also be employed.

Subsequent to the dissolving or dispersing or suspending, an appropriate acid is added in order to allow for the formation of the salt. Said acid may also be dissolved or dispersed or suspended in one or more of the above defined solvents.

The precipitated and/or crystallized salt may be separated off from the reaction mixture by filtration.

Solvent adhering to the precipitate may be removed by drying and/or in vacuo.

In one embodiment, the employed acid is hydrochloric acid or methane sulphonic acid, and the resulting salt is the chloride or the mesylate. The melting point of the mesylate is 173.1 °C as determined by differential scanning calorimetry employing a heating rate of 10 K min⁻¹.

In another embodiment, the employed acid is hydrobromic acid, or acetic acid, or citric acid, or maleic acid, or succinic acid, and the resulting salt is the bromide, or the acetate (m.p. 142.2 °C), or the mono citrate (m.p. 151.5 °C), or the mono maleinate (m.p. 160.1 °C), or the mono succinate (m.p. 177.2 °C).

In one embodiment, the yield of salt is at least 95 % by weight having a purity of at least 98.5 % by weight.

In one embodiment, the purity is at least 99.9 % by weight.

In one embodiment, the overall yield of the reaction sequence comprising steps (i) to (v) is at least 65 % by weight.

Salts of 1-amino-1,3,3,5,5-pentamethylcyclohexane may exist in polymorphic or pseudopolymorphic forms.

The term *polymorphism"* defines the ability of a solid material to exist in more than one form or crystal structure.

The term *pseudopolymorphism"* defines the ability of a solid material to form different crystal types as the result of hydration or solvation.

Neramexane hydrochloride may exist in two polymorphic forms and three pseudopolymorphic hydrate forms.

For the purpose of this disclosure, the two polymorphic forms are termed form A and form E.

For the purpose of this disclosure, the three pseudopolymorphic forms are the monohydrate form termed as form B, the sesquihydrate form termed as form C and the trihydrate form termed as form D.

In one embodiment, form A may be prepared by drying neramexane hydrochloride at about 50 °C/100 mbar. In one embodiment, form A may contain water in an amount up to approx. 0.7 % by weight. If the form is completely dried, it is termed for the purpose of this disclosure form A'.

Forms A and E are related enantiotropically, i.e. they may be reversibly transformed into each other by changing the temperature. The low-temperature form A (melting point. 221 °C) is thermodynamically stable up to at least 70 °C. Above 70 °C it is transferred into the high-temperature form E (m.p. 241 °C).

At 25 °C, form A may be transformed into the hydrates at above approx. 50 % relative humidity (r.h.). Form C is the most stable form of the pseudopolymorphs. At 25 °C and below approx. 25 % r.h. form C may be transformed into form A and at 40 °C below approx. 33 % r.h. The next stable hydrate is form B. Form D is stable only as a suspension in water.

In one aspect, the invention relates to 1-amino-1,3,3,5,5-pentamethylcyclohexane hydrochloride form A, or form A', or form E.

In another aspect, the invention relates to 1-amino-1,3,3,5,5-pentamethylcyclohexane hydrochloride form B, or form C, or form D.

In another embodiment, the invention relates to a mixture of at least two or more of any of said forms.

The polymorphs and pseudopolymorphs may be characterized by X-ray powder diffraction. Samples of forms A, B and D generally exhibit three to four strong peaks. Ground samples show remarkable variations in peak intensity compared to unground samples.

In one embodiment, the method comprises the following steps (i) to (iv):
(i) converting isophorone to 3,3,5,5-tetramethylcyclohexanone in the presence of methylmagnesium chloride copper(I) iodide, lithium chloride and tetrahydrofurane;
(ii) converting 3,3,5,5-tetramethylcyclohexanone as obtained in step (i) to 1-hydroxy-1,3,3,5,5-pentamethylcyclohexane in the presence of methylmagnesium chloride and tetrahydrofurane;
(iii) converting 1-hydroxy-1,3,3,5,5-pentamethylcyclohexane as obtained in step (ii) to 1-chloroacetamido-1,3,3,5,5-pentamethylcyclohexane in the presence of chloroacetonitrile, acetic acid and sulphuric acid;
(iv) converting 1-chloroacetamido-1,3,3,5,5-pentamethylcyclohexane as obtained in step (iii) to 1-amino-1,3,3,5,5-pentamethylcyclohexane in the presence of thiourea, water and hydrochloric acid.

In one embodiment, the method comprises the additional step (v):
(v) converting 1-amino-1,3,3,5,5-pentamethylcyclohexane as obtained in step (iv) to a pharmaceutically acceptable salt thereof by adding methane sulphonic acid.

*1-amino-1-ethyl-3,3,5,5-tetramethylcyclohexane and 1-amino-3-ethyl-1,3,5,5-tetramethylcyclohexane as side-products*

In one embodiment of the reaction sequence according to steps (i) to (iv), respectively according to steps (i) to (v), wherein the conversion according to step (i) is effected by using a methylmagnesium Grignard reagent such as methylmagnesium chloride, besides 1-amino-1,3,3,5,5-pentamethylcyclohexane, respectively a salt of 1-amino-1,3,3,5,5-pentamethylcyclohexane, further amino compounds may be formed, which are different from the target compound 1-amino-1,3,3,5,5-pentamethylcyclohexylamine or the respective salt thereof.

In one embodiment, three side products may be formed. They may e.g. detected by gas chromatographical analysis.

In one embodiment, 1-amino-3-ethyl-1,3,5,5-tetramethylcyclohexane may be formed as a side-product. Since this compound has two chiral centers, two diastereomers may be detected.

In one embodiment, 1-amino-1-ethyl-3,3,5,5-tetramethylcyclohexane is additionally formed.

In one embodiment, the occurrence of 1-amino-3-ethyl-1,3,5,5-tetramethylcyclohexane may be attributed to the addition of an ethyl group instead of a methyl group to isophorone in step (i) to yield the respective cyclohexanone. If subsequent to the addition the sequence analogous to steps (ii) to (iv), respectively analogous to steps (ii) to (v) is performed, said amine, respectively a salt thereof, is formed.

In one embodiment, the occurrence of 1-amino-1-ethyl-3,3,5,5-tetramethylcyclohexane may be attributed to the addition of an ethyl group instead of a methyl group to the carbonyl group of the respective cyclohexanone in step (ii). If subsequent to the addition the sequence analogous to steps (iii) to (iv), respectively steps (iii) to (v) is performed, said amine, respectively a salt thereof, is formed.

In one embodiment, the occurrence of said side-products may be attributed to the contamination of the employed methylmagnesium Grignard reagent with an ethylmagnesium Grignard reagent.

In one embodiment, the occurrence of said undesired side-products may be suppressed by employing a purified methylmagnesium Grignard reagent which is free of an ethylmagnesium Grignard reagent such as ethylmagnesium chloride.

In one embodiment, methylmagnesium chloride contains less than 1 % by weight ethylmagnesium chloride based on the total amount of methylmagnesium chloride and ethylmagnesiumchloride, or less than 0.5 % by weight, or less than 0.1 % by weight.

In one embodiment, undesired side-products may be removed from the target product by purifying the amine obtained according to step (iv). In one embodiment, the amine may be purified by distillation, wherein the side-products are removed.

In another embodiment, the salt obtained according to step (v) is purified. In one embodiment, said salt may be purified by a step of re-crystallization. A suitable solvent is e.g. a solvent selected from the solvents as used in step (v). In one embodiment, the solvent is anisole. In one embodiment, the salt is the mesylate.

With reference to the reaction scheme as referred to in the Background section, the method according to the invention allows the omission of complex cleaning steps of the intermediates **2, 3** and **6** such as distillation or recrystallization or chromatography, which result in product loss. The novel method provides Neramexane or a pharmaceutically acceptable salt thereof in a yield of at least 60 % by weight in acceptable purity. Accordingly, the novel simplified method of producing Neramexane may be performed on an advantageous economical industrial scale.

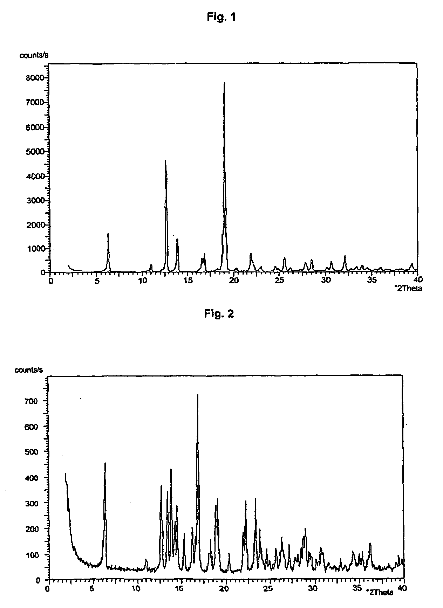

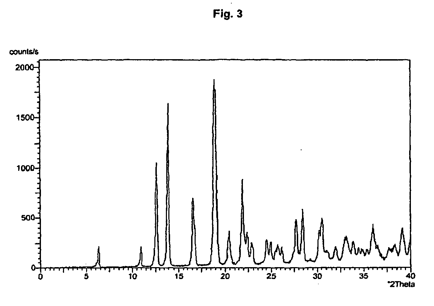

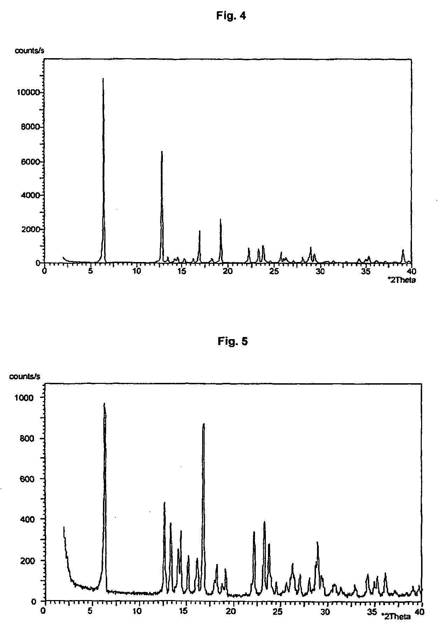

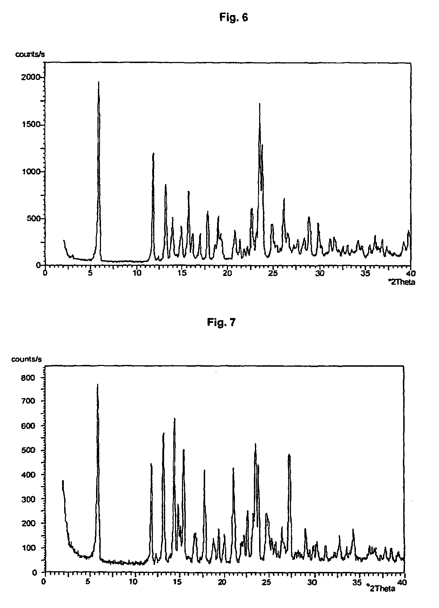

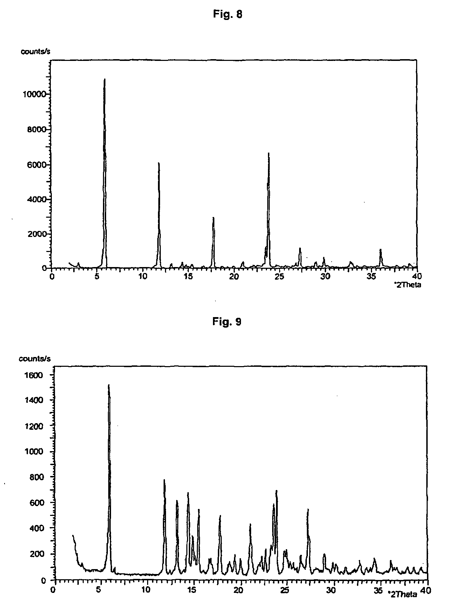

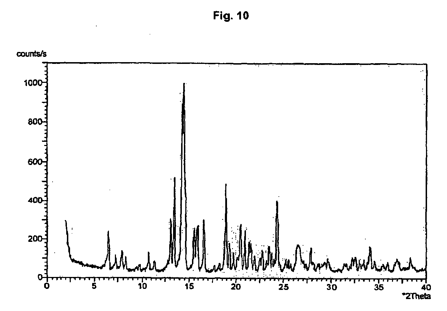

Figure 1 to 10 exibit X-ray powder diffraction diagrams of forms A, A', B, C, D, and E. The x-axis shows 20 [deg] / d [A], the y-axis the intensity in arbitrary units.
Figure 1: Form A
Figure 2: Form A ground
Figure 3: Form A'
Figure 4: Form B
Figure 5: Form B ground
Figure 6: Form C
Figure 7: Form C ground
Figure 8: Form D
Figure 9: Form D ground
Figure 10: Form E

### EXAMPLES

### Example 1

A mixture of 93 g methylmagnesium chloride and 372 g tetrahydrofurane is added by dropping to a stirred mixture of 139 g isophorone, 19 g copper(I) iodide, 8.4 g lithium chloride and 1,550 g tetrahydrofurane, wherein the inorganic compounds have been dissolved prior to the dropping. The dropping rate is selected such that the temperature of the mixture can be kept between 5 and 15 °C. After the addition is terminated, the mixture is stirred for 60 minutes. Subsequently, diluted hydrochloric acid is added to decompose an excess of methylmagnesium chloride, and to decompose basic magnesium compounds. The mixture is extracted twice with petroleum ether. The extracts are combined and washed with ammonia. Subsequently, the solvent is distilled off. The yield of crude target compound is quantitative (153 g). The content of 3,3,5,5-tetramethylcyclohexanone in the crude product is about 91 % by weight as determined by gas-liquid chromatography. The crude product contains approximately 2 % by weight non-reacted isophorone, less than 1 % by weight 1,3,5,5-tetramethylcyclohexanol generated by 1,2-additon of the Grignard reagent to isophorone, or olefins generated from said compound, and 1 % by weight 1-hydroxy-1,3,3,5,5-pentamethylcyclohexane.

### Example 2

A mixture of 153 g 3,3,5,5-tetramethylcyclohexanone as obtained in Example 1 and 153 g tetrahydrofurane is dropped to a stirred mixture of 93 g methylmagnesium chloride and 372 g tetrahydrofurane. The dropping rate is selected such that the temperature of the mixture can be kept between 5 and 15 °C. After the addition is terminated, the mixture is stirred for 60 minutes. Subsequently, diluted hydrochloric acid is added to decompose an excess of methylmagnesium chloride, and to decompose basic magnesium compounds. The mixture is extracted twice with petroleum ether. The extracts are combined and the solvent is distilled off. The crude yield of 1-hydroxy-1,3,3,5,5-pentamethylcyclohexane is quantitative (170 g). The content of target compound in the crude product is about 95 % by weight as determined by gas-liquid chromatography.

### Example 3

294 g concentrated sulphuric acid are dropped to a stirred mixture of crude 170 g 1-hydroxy-1,3,3,5,5-pentamethylcyclohexane as obtained in Example 2, 150 g chloroacetonitrile and 320 g glacial acetic acid. The dropping rate is selected such that the temperature of the reaction mixture could be kept between 5 and 10 °C. After the dropping is terminated, the mixture is stirred for another 60 minutes. Subsequently, the mixture is poured onto a mixture of ice and water. The precipitating target compound 1-chloroacetamido-1,3,3,5,5-pentamethylcyclohexane is separated off by filtration. After drying, 230 g target compound are obtained. The yield is nearly quantitative (94 %).

### Example 4

A mixture of 245 g 1-chloroacetamido-1,3,3,5,5-pentamethylcyclohexane as prepared according to Example 3, 91 g thiourea, 2,700 g water and 220 g hydrochloric acid (33 % acid) is heated under reflux. After a reaction time of 6 hours, the mixture is cooled down to ambient temperature, and the pH of the mixture is set to a value of at least 7 by adding sodium hydroxide. Subsequently, the mixture is extracted twice with petroleum ether. The extracts are combined. After distilling petroleum ether off, crude 1-amino-1,3,3,5,5-pentamethylcyclohexane is obtained in a yield of 97 % (159 g). The crude product had a content of target compound of 97 % by weight as determined by gas-liquid chromatography. The product is purified by distillation.

### Example 5

101 g methane sulphonic acid are dropped to a mixture of 169 g 1-amino-1,3,3,5,5-pentamethylcyclohexane as obtained in Example 4 in 1,860 g ethyl acetate, so that the temperature can be kept between 0 and 5 °C. After stirring the mixture for 60 minutes, the precipitate is filtered off, washed with ethyl acetate and dried in vacuo. The product yield is 241 g (91 % by weight).

### Example 6

1-amino-1,3,3,5,5-pentamethylcyclohexane hydrochloride is prepared by precipitating the salt with hydrochloric acid in ethylmethylketone. The precipitated salt is filtered off and dried at 50 °C/100 mbar to afford form A.

1-amino-1,3,3,5,5-pentamethylcyclohexane hydrochloride is prepared by precipitating the salt with hydrochloric acid in ethylmethylketone. The precipitated salt is filtered off and air dried to afford form B.

1 g of form A is stirred as suspension in 10 ml acetone and 0.5 ml water at room temperature for 24 h. The product is filtered and dried in an air stream (24 °C, 40 % r.h.) for 1 to 2 min to afford form C.

1 g of form A is dissolved in 5 ml water at 70 °C. At room temperature, the product crystallizes to afford form D.

1 g of form A is heated in a Schlenk tube under argon to approx. 230 °C. This temperature is maintained for 15 min. After cooling to room temperature, the sample is stored under inert atmosphere to afford Form E.

## Claims

1. Method of preparing *1-amino-1,3,3,5,5-pentamethylcyclohexane* or a pharmaceutically acceptable salt thereof, comprising at least steps (i) to (iv):
(i) converting isophorone to 3,3,5,5-tetramethylcyclohexanone;
(ii) converting 3,3,5,5-tetramethylcyclohexanone as obtained in step (i) to 1-hydroxy-1,3,3,5,5-pentamethylcyclohexane;
(iii) converting 1-hydroxy-1,3,3,5,5-pentamethylcyclohexane as obtained in step (ii) to 1-chloroacetamido-1,3,3,5,5-pentamethylcyclohexane;
(iv) converting 1-chloroacetamido-1,3,3,5,5-pentamethylcyclohexane as obtained in step (iii) to 1-amino-1,3,3,5,5-pentamethylcyclohexane;
wherein at least one of 3,3,5,5-tetramethylcyclohexanone, 1-hydroxy-1,3,3,5,5-pentamethylcyclohexane, 1-chloroacetamido-1,3,3,5,5-pentamethylcyclohexane, is not subjected to a purification step.

2. Method according to claim 1, wherein the conversion in step (i) is effected by the reaction of isophorone with methylmagnesium chloride in the presence of a copper(I) halide and a lithium halide.

3. Method according claim 1 or 2, wherein the conversion in step (ii) is effected by the reaction of 3,3,5,5-tetramethylcyclohexanone with methylmagnesium chloride.

4. Method according to any one of the preceding claims, wherein the conversion in step (iii) is effected by the reaction of 1-hydroxy-1,3,3,5,5-pentamethylcyclohexane with chloroacetonitrile in acidic solution.

5. Method according to any one of the preceding claims, wherein the conversion in step (iv) is effected by reacting a mixture comprising 1-chloracetamido-1,3,3,5,5-pentamethylcyclohexane, thiourea and water.

6. Method according to any one of the preceding claims, further comprising step (v):
(v) converting 1-amino-1,3,3,5,5-pentamethylcyclohexane as obtained in step (iv) to a pharmaceutically acceptable salt thereof;

7. Method according to claim 6, wherein the conversion in step (v) is effected by the reaction of 1-amino-1,3,3,5,5-pentamethylcyclohexane with an acid.

8. Method according to claim 7, wherein the acid is methane sulphonic acid.

9. Method according to any one of the preceding claims, comprising
(i) converting isophorone to 3,3,5,5-tetramethylcyclohexanone in the presence of methylmagnesium chloride copper(I) iodide, lithium chloride and tetrahydrofurane;
(ii) converting 3,3,5,5-tetramethylcyclohexanone obtained in step (i) to 1-hydroxy-1,3,3,5,5-pentamethylcyclohexane in the presence of methylmagnesium chloride and tetrahydrofurane;
(iii) converting 1-hydroxy-1,3,3,5,5-pentamethylcyclohexane obtained in step (ii) to 1-chloroacetamido-1,3,3,5,5-pentamethylcyclohexane in the presence of chloroacetonitrile, acetic acid and sulphuric acid;
(iv) converting 1-chloroacetamido-1,3,3,5,5-pentamethylcyclohexane obtained in step (iii) to 1-amino-1,3,3,5,5-pentamethylcyclohexane in the presence of thiourea, water and hydrochloric acid.

10. Method according to any one of the preceding claims, wherein said methylmagnesium chloride is free of ethylmagnesium chloride.

## Patentansprüche

1. Verfahren zur Herstellung von 1-Amino-1,3,3,5,5-pentamethylcyclohexan oder eines pharmazeutisch verträglichen Salzes davon, umfassend mindestens Stufen
(i) to (iv):
(i) Überführen von Isophoron in 3,3,5,5-Tetramethylcyclohexanon;
(ii) Überführen von 3,3,5,5-Tetramethylcyclohexanon wie erhalten in Stufe (i) in 1-Hydroxy-1,3,3,5,5-pentamethylcyclohexan;
(iii) Überführen von 1-Hydroxy-1,3,3,5,5-pentamethylcyclohexan wie erhalten in Stufe (ii) in 1-Chloracetamido-1,3,3,5,5-pentamethylcyclohexan;
(iv) Überführen von 1-Chloracetamido-1,3,3,5,5-pentamethylcyclohexan wie erhalten in Stufe (iii) in 1-Amino-1,3,3,5,5-pentamethylcyclohexan;
wobei mindestens eine der Verbindungen 3,3,5,5-Tetramethylcyclohexanon, 1-Hydroxy-1,3,3,5,5-pentamethylcyclohexan, 1-Chloracetamido-1,3,3,5,5-pentamethylcyclohexan keinem Reinigungsschritt unterzogen wird.

2. Verfahren nach Anspruch 1, wobei die Überführung in Stufe (i) bewirkt wird durch die Reaktion von Isophoron mit Methylmagnesiumchlorid in der Anwesenheit eines Kupfer(I)halogenids und einem Lithiumhalogenid.

3. Verfahren nach Anspruch 1 oder 2, wobei die Überführung in Stufe (ii) bewirkt wird durch die Reaktion von 3,3,5,5-Tetramethylcyclohexanon mit Methylmagnesiumchlorid.

4. Verfahren nach einem der vorstehenden Ansprüche, wobei die Überführung in Stufe (iii) bewirkt wird durch die Reaktion von 1-Hydroxy-1,3,3,5,5-pentamethylcyclohexan mit Chloracetonitril in saurer Lösung.

5. Verfahren nach einem der vorstehenden Ansprüche, wobei die Überführung in Stufe (iv) bewirkt wird durch Reaktion einer Mischung umfassend 1-Chloracetamido-1,3,3,5,5-pentamethylcyclohexan, Thioharnstoff und Wasser.

6. Verfahren nach einem der vorstehenden Ansprüche, weiter umfassend Stufe (v):
(v) Überführen von 1-Amino-1,3,3,5,5-pentamethylcyclohexan wie erhalten in Stufe (iv) in ein pharmazeutisch verträgliches Salz davon.

7. Verfahren nach Anspruch 6, wobei die Überführung in Stufe (v) bewirkt wird durch die Reaktion von 1-Amino-1,3,3,5,5-pentamethylcyclohexan mit einer Säure.

8. Verfahren nach Anspruch 7, wobei die Säure Methansulfonsäure ist.

9. Verfahren nach einem der vorstehenden Ansprüche, umfassend:
(i) Überführen von Isophoron in 3,3,5,5-Tetramethylcyclohexanon in Gegenwart von Methylmagnesiumchlorid, Kupfer(I)iodid, Lithiumchlorid und Tetrahydrofuran;
(ii) Überführen von 3,3,5,5-Tetramethylcyclohexanon wie erhalten in Stufe (i) in 1-Hydroxy-1,3,3,5,5-pentamethylcyclohexan in Gegenwart von Methylmagnesiumchlorid und Tetrahydrofuran;
(iii) Überführen von 1-Hydroxy-1,3,3,5,5-pentamethylcyclohexan wie erhalten in Stufe (ii) in 1-Chloracetamido-1,3,3,5,5-pentamethylcyclohexan in Gegenwart von Chloracetonitril, Essigsäure und Schwefelsäure;
(iv) Überführen von 1-Chloracetamido-1,3,3,5,5-pentamethylcyclohexan wie erhalten in Stufe (iii) in 1-Amino-1,3,3,5,5-pentamethylcyclohexan in Gegenwart von Thioharnstoff, Wasser und Salzsäure.

10. Verfahren nach einem der vorstehenden Ansprüche, wobei besagtes Methylmagnesiumchlorid frei von Ethylmagnesiumchlorid ist.

## Revendications

1. Procédé de préparation de 1-amino-1,3,3,5,5-pentaméthylcyclohexane ou d'un sel pharmaceutiquement acceptable de celui-ci, comprenant au moins les étapes (i) à (iv) :
(i) de conversion d'isophorone en 3,3,5,5-tétraméthylcyclohexanone ;
(ii) de conversion de 3,3,5,5-tétraméthylcyclohexanone comme obtenue dans l'étape (i) en 1-hydroxy-1,3,3,5,5-pentaméthylcyclohexane ;
(iii) de conversion de 1-hydroxy-1,3,3,5,5-pentaméthylcyclohexane comme obtenu dans l'étape (ii) en 1-chloroacétamido-1,3,3,5,5-pentaméthylcyclohexane ;
(iv) de conversion de 1-chloroacétamido-1,3,3,5,5-pentaméthylcyclohexane comme obtenu dans l'étape (iii) en 1-amino-1,3,3,5,5-pentaméthylcyclohexane ;
dans lequel au moins un parmi la 3,3,5,5-tétraméthylcyclohexanone, le 1-hydroxy-1,3,3,5,5-pentaméthylcyclohexane, le 1-chloroacétamido-1,3,3,5,5-pentaméthylcyclohexane, n'est pas soumis à une étape de purification.

2. Procédé selon la revendication 1, dans lequel la conversion dans l'étape (i) est réalisée par la réaction d'isophorone avec du chlorure de méthylmagnésium en présence d'un halogénure de cuivre (I) et d'un halogénure de lithium.

3. Procédé selon la revendication 1 ou 2, dans lequel la conversion dans l'étape (ii) est réalisée par la réaction de 3,3,5,5-tétraméthylcyclohexanone avec du chlorure de méthylmagnésium.

4. Procédé selon l'une quelconque des revendications précédentes, dans lequel la conversion dans l'étape (iii) est réalisée par la réaction de 1-hydroxy-1,3,3,5,5-pentaméthylcyclohexane avec du chloroacétonitrile dans une solution acide.

5. Procédé selon l'une quelconque des revendications précédentes, dans lequel la conversion dans l'étape (iv) est réalisée par réaction d'un mélange comprenant du 1-chloroacétamido-1,3,3,5,5-pentaméthylcyclohexane, de la thiourée et de l'eau.

6. Procédé selon l'une quelconque des revendications précédentes, comprenant de plus l'étape (v) :
(v) de conversion de 1-amino-1,3,3,5,5-pentaméthylcyclohexane comme obtenu dans l'étape (iv) en un sel pharmaceutiquement acceptable de celui-ci.

7. Procédé selon la revendication 6, dans lequel la conversion dans l'étape (v) est réalisée par la réaction de 1-amino-1,3,3,5,5-pentaméthylcyclohexane avec un acide.

8. Procédé selon la revendication 7, dans lequel l'acide est l'acide méthanesulfonique.

9. Procédé selon l'une quelconque des revendications précédentes, comprenant
(i) la conversion d'isophorone en 3,3,5,5-tétraméthylcyclohexanone en présence de chlorure de méthylmagnésium, d'iodure de cuivre (I), de chlorure de lithium et de tétrahydrofurane ;
(ii) la conversion de 3,3,5,5-tétraméthylcyclohexanone obtenue dans l'étape (i) en 1-hydroxy-1,3,3,5,5-pentaméthylcyclohexane en présence de chlorure de méthylmagnésium et de tétrahydrofurane ;
(iii) la conversion de 1-hydroxy-1,3,3,5,5-pentaméthylcyclohexane obtenu dans l'étape (ii) en 1-chloroacétamido-1,3,3,5,5-pentaméthylcyclohexane en présence de chloroacétonitrile, d'acide acétique et d'acide sulfurique
(iv) la conversion de 1-chloroacétamido-1,3,3,5,5-pentaméthylcydohexane obtenu dans l'étape (iii) en 1-amino-1,3,3,5,5-pentaméthylcyclohexane en présence de thiourée, d'eau et d'acide chlorhydrique.

10. Procédé selon l'une quelconque des revendications précédentes, dans lequel ledit chlorure de méthylmagnésium est exempt de chlorure d'éthylmagnésuim.
